# EUROPÄISCHE PATENTANMELDUNG

(11) **EP 1 764 042 A2**
(43) Date of publication of application: **21.03.2007**
(21) Application number: 06019063.4
(22) Date of filing: 12.09.2006
(51) Int. Cl.: A61B 17/064

(54) **Compression staple**

(30) Priority: 12.09.2005 US 715613 P
(71) Applicant: Arthrex, Inc., Naples, Florida 34108-1945 (US)
(72) Inventor: Beckendorf, Brandon, Naples Florida 34108-1945 (US); Albertorio, Ricardo, Naples Florida 34119 (US); Cicchinelli, Luke D., Greenville North Carolina 27858 (US)
(74) Representative: Siniscalco, Fabio

(57) **Abstract**

A compression staple (100) that can secure tissue (for example, bone) easily and effectively, and can provide improved fixation capabilities. The compression staple comprises a yolk or central portion (20) having an S-shaped profile and two posts (50) located at the bottom surface of the central portion (20). The two posts (50) have a cylindrical configuration and may be cannulated. The posts (50) may be also provided with a plurality of ribbed barbs (55), preferably extending about one third the length of the posts. The compression staple (100) may additionally comprise two spikes (80) located at the bottom surface of the central portion (20). The spikes (80) are configured to increase fixation of the staple (100) into the bone and to allow easy penetration through the cortical bone into the cancellous bone.

## Description

### FIELD OF THE INVENTION

The present invention is directed to a compression staple for attachment of bone to bone.

### BACKGROUND OF THE INVENTION

Treatment of a bone fracture typically involves fastening of one bone segment to the other, to stabilize and immobilize the bones for the duration of the bone consolidation process. Traditionally, bone fixation has been accomplished by a variety of devices and techniques, the more common involving the use of metallic fastening devices such as screws, connector plates, pins and clips. These methods involve the drilling of screw holes in the bone and the use of related equipment such as drill hole templates.

Conventional U-shaped clips have been used for treating bone fractures, the clip legs being installed one each in holes in the opposing bone segments. The rigid structure of such clips provides rigid immobilization of the fracture zone. Although devices such as the U-shaped clips maintain the distance between bone segments, they hinder compression induced by contractions of skeletal muscles in some cases, and prevent the establishment of compressive force between the bone segments which is favorable to bone consolidation.

A number of fastening devices have been designed to deliver compression. For example, U.S. Pat. No. 3,939,294 describes a clasp of spring material having a pair of spaced-apart, inwardly inclined legs connected by a Z-shaped upper portion. Sloped holes are drilled in adjoining bone segments and tools are used to manipulate and install one leg, so that the other leg is subsequently pulled toward the other hole (spreading the Z-shaped elastic portion) and then inserted in the other hole. Unfortunately, this method requires drilling of specially sloped holes, involves multiple steps and is time-consuming, and requires a relatively large surgical opening. Also, the manual installation of the clasp using hemostats and similar devices is difficult and requires meticulous skill and handling.

U.S. Pat. No. 5,660,188 also requires precise manual installation of two legs of a clip in pre-drilled holes. The clip has a bridge of two side-by-side crimpable elements, and the jaws of a crimping tool must be used on the embedded clip to deformingly spread apart these elements, causing the legs to draw to each other. This technique involving crimpable dips is imprecise in setting up suitable compressive forces, requires hole drilling and related problems, and does not minimize the size of the surgical opening.

In view of the limitations of the above-mentioned methods, stapling has been proposed as an effective way for fastening bone segments and producing compression. For example, in U.S. Pat. Nos. 5,053,038 and 5,662,655, "compression" staples are applied to the bone by a stapler. These staples have legs shaped with beveled ends and/or have divergent legs that will be forced apart from each other during implantation, which flexes springy upper parts of the legs therefore tending to set up compression. Unfortunately, trauma to the bone and even fracture may occur due to driving of the compound-shaped legs into the bone mass. As the legs are typically rectangular, they create high-stress concentration around the corners of the legs during the insertion and, thus, the amount of stress resulting from the compression of the staple is not evenly displaced around the perimeter of the rectangular legs. As a result, unwanted bone cracks and fractures occur upon the insertion of the staple.

Accordingly, a need exists for an improved compression staple for bone fixation that can be installed easily and effectively, and yet can provide improved fixation capabilities. A need also exists for a fixation device having improved structural properties.

### SUMMARY OF THE INVENTION

The present invention overcomes disadvantages of the prior art and fulfills the needs discussed above by providing a compression staple that can secure tissue (for example, bone) easily and effectively, and can provide improved fixation capabilities.

The compression staple comprises a yolk or central portion having an S-shaped profile and two posts located at the bottom surface of the central portion. The two posts have a cylindrical configuration and may be cannulated. The posts may be also provided with a plurality of ribbed barbs, preferably extending about one third the length of the posts. If barbs are provided, the barbs may additionally be configured with undercuts to promote fixation of the staple into the bone and to prevent pullout of the staple.

The compression staple may also comprise two spikes located at the bottom surface of the central portion. The spikes are configured to increase fixation of the staple into the bone and to allow easy penetration through the cortical bone into the cancellous bone. After the staple is fully inserted, the spikes ensure that shearing of the bone resulting from normal forces is minimized. The spikes are positioned such that after the staple is compressed, they lie outside of the fracture site.

These and other features and advantages of the invention will be more apparent from the following detailed description that is provided in connection with the accompanying drawings and illustrated exemplary embodiments of the invention.

### BRIEF DESCRIPTION OF THE DRAWINGS

FIG.1 is a top view of a compression staple of the present invention;

FIG. 2 is an isometric view of the compression staple of FIG. 1;

FIG. 3 is a front view of the compression staple of FIG.1;

FIG. 4 is a side view of the compression staple of FIG. 1; and

FIG. 5 is another top view of the compression staple of FIG. 1 illustrating the compression force applied to the staple.

### DETAILED DESCRIPTION OF THE PREFERRED EMBODIMENT

The following description is provided to enable any person skilled in the art to make and use the invention and sets forth the best modes contemplated by the inventors of carrying out their invention. Various modifications, however, will remain readily apparent to those skilled in the art.

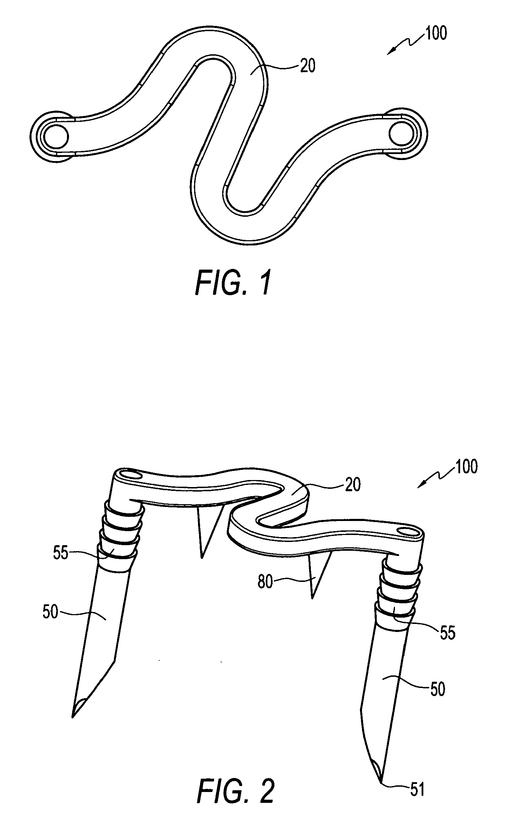

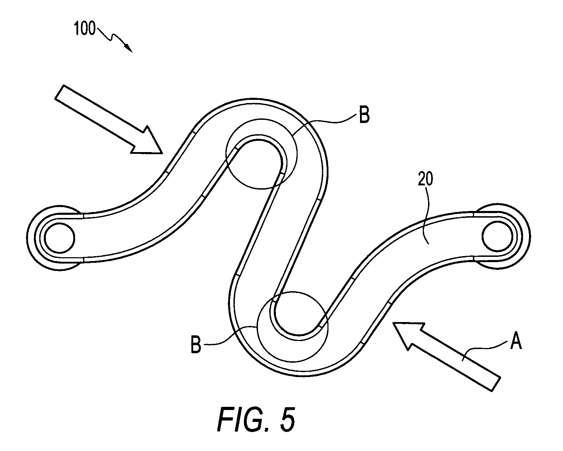

FIGS. 1-5 illustrate a compression staple 100 according to the present invention. As shown in FIGS. 1-3, the compression staple 100 comprises a central portion 20 having an S-shaped profile and two posts or legs 50 located at the bottom surface of the central portion. The S-shaped configuration of the central portion 20 provides maximum compression at a minimal material yield point while reducing the torque on the staple posts 50. As compression is a function of the distance between the staple posts before and after compression of the staple, compression is applied at the sides of the staple illustrated by arrows A in FIG. 5. The compression applied on the sides reduces the moment of the staple legs 50, thus reducing the amount of shear of the two bone segments fused during compression.

The two cutouts B illustrated by the circles in FIG. 5 also denote the cross-section gradually becoming smaller as the staple profile bends 180 degrees. This feature reduces the stress at this point while minimizing the amount of force it takes to make the material yield. Finite element analysis show that regions B are the highest regions of stress of the staple during compression.

As shown in FIG. 2, the two posts 50 have a cylindrical configuration and are preferably cannulated. Although reference to posts 50 will be made in this application as to the cannulated posts 50, it must be understood that the invention is not limited to this embodiment and also contemplates posts or legs which are not cannulated. The two posts 50 may be also provided with a plurality of barbs 55 extending about one third the length of the posts. Preferably, the barbs are provided with undercuts to promote fixation of the staple 100 into the tissue and to prevent pullout of the staple. These features allow a surgeon to insert the staple roughly 2/3 down into the bone (denoted by the beginning of the barbs) as desired by the technique, and then compress the staple. The slight amount of torque resulting from compressing the staple is easily displaced by the cylindrical cannulated legs 50, which allow the entire staple to rotate during compression instead of just the staple legs.

If the surgeon is not satisfied with the resulting compression after the staple is fully compressed, the surgeon may simply remove the staple by pulling it straight out. If the surgeon is satisfied with the resulting compression, the staple may be fully inserted (tamped) into place. The barbs provide anchorage of the staple into both the cortical bone as well as the cancellous bone, which will prevent the staple from backing out.

Because the cross-sectional area of the staple legs is reduced, the cylindrical legs allow the staple to be inserted easier than a conventional staple with rectangular or square legs. Also, the cylindrical legs decrease the likelihood of hard bone cracking upon insertion of the staple, as high stress concentration areas around the cylindrical legs are greatly reduced.

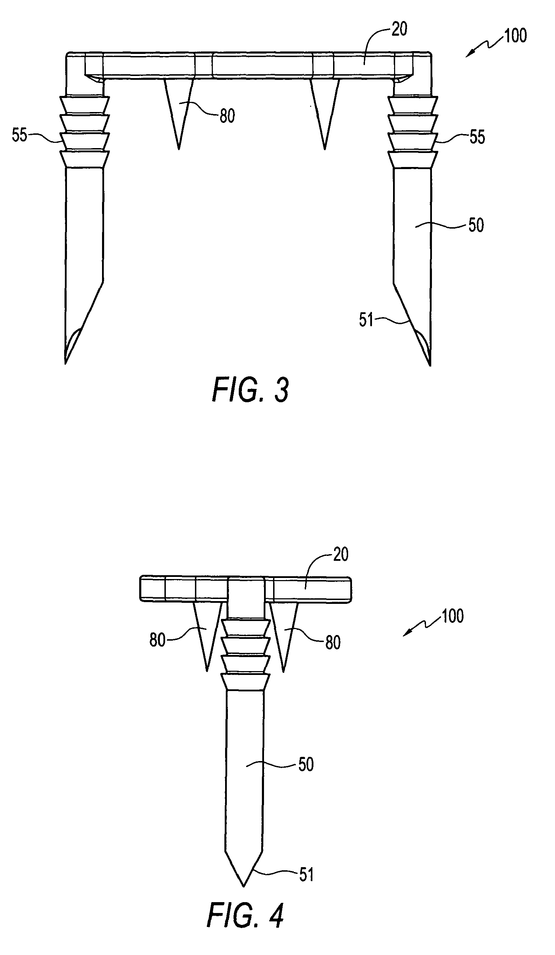

As shown in FIGS. 2 and 3, the two cannulated posts 50 are provided with trocar tips 51 which ensure that the staple cuts through the bone during insertion without fracturing the bone. The configuration of the trocar tip 51 also allows staple 100 to provide some compression, even without manually compressing the top of the staple. The angled tip forces bone towards fracture/joint line, thus creating compression as it is tamped into place.

As shown in FIGS. 2-4, compression staple 100 also comprises two spikes 80 located at the bottom surface of the central portion 20. The spikes 80 are configured to increase fixation of the staple into the bone and to allow easy penetration through the cortical bone into the cancellous bone. After the staple is fully inserted, the spikes ensure that shearing of the bone resulting from normal forces is minimized. As shown in FIG. 4, the spikes are laterally located relative to the longitudinal axis of the central portion 20 of the staple and of the two posts 50. In this manner, and as a result of the positioning of the spikes, after the staple is compressed the spikes lie just outside of the fracture site.

Preferably, the compression staple 100 is manufactured from stainless steel, which provides enough strength for the staple leg / "S" body joint under compression (minimizes any bowing outward of the legs), while having a low enough yield point so that the staple may permanently compress and thus undergo plastic deformation. The compression staple 100 may be also manufactured from conventional implant metals such as titanium and/or may comprise a shape memory material or alloy such as nickel titanium, to enhance fixation.

The present invention also provides a method for attaching tissue to tissue (for example, bone to bone, or soft tissue to bone) by employing a compression staple that can secure tissue easily and effectively without sutures. The method of the present invention comprises the steps of: (i) providing a compression staple having a central portion with an S-shaped configuration and two posts located at the bottom surface of the central portion; and (ii) attaching a first tissue region to a second tissue region by inserting at least one of the two posts within at least one of the first and second tissue regions.

In an exemplary embodiment, the compression staple can secure a first bone fragment to a second bone fragment of a fracture fusion site. The method for installing the compression staple 100 of the present invention comprises the steps of: (i) compressing the fracture fusion site with a hand clap; (ii) using the desired staple size as a positional reference, driving a first guide pin (for example, a K wire) into the bone and subsequently inserting one of the two cannulated posts over the guide pin; (iii) aligning the staple to the desired position, and placing a second guide pin (for example, a K wire) through the second cannulated post; (iv) driving the second guide pin into the bone; (v) using a mallet and tamp, for example, partially impacting the staple into the bone until the base of the lowest barb on the cannulated posts of the staple reaches the bone surface; (vi) using a compression device, such as a stationary forked device, applying compression force across the "S" of the staple to twist or torque the "S" of the staple into compression; and (vii) fully inserting the staple into the bone so that the staple is flush with the bone surface.

The above description and drawings illustrate preferred embodiments which achieve the objects, features and advantages of the present invention. It is not intended that the present invention be limited to the illustrated embodiments. Any modification of the present invention which comes within the spirit and scope of the following claims should be considered part of the present invention.

## Claims

1. A compression staple, comprising:
a body having an S-shaped configuration, a first surface, a second surface, and a middle region;
a plurality of posts extending from the first surface of the body; and
a plurality of spikes extending from the first surface of the body and located in the middle region of the body.

2. The compression staple of claim 1, wherein each of the plurality of posts is located at a proximal end and at a distal end, respectively, of the body.

3. The compression staple of claim 1, wherein the posts have a first length, and the spikes have a second length, the first length being greater than the second length.

4. The compression staple of claim 1, wherein the posts are cannulated.

5. The compression staple of claim 1, wherein the posts have a cylindrical cross-sectional configuration.

6. The compression staple of claim 1, wherein the body of the staple has sides that form an angle of about ninety degrees relative to at least one of the first and second surfaces.

7. The compression staple of claim 1, wherein the posts are provided with a plurality of ribbed barbs that extend about one third the length of the posts.

8. A compression staple, comprising:
a body having an S-shaped profile, a top surface, a bottom surface, a side surface forming an angle of about ninety degree with at least one of the top surface and the bottom surface, and a middle region;
a plurality of cylindrical cannulated posts extending from the bottom surface of the body, each of the plurality of cylindrical cannulated posts being located at opposing ends of the body; and
a plurality of spikes extending from the bottom surface of the body and located in the middle region of the body.

9. The compression staple of claim 8, wherein at least one of the plurality of posts is provided with a plurality of ribbed barbs that extend about one third the length of the posts.

10. The compression staple of claim 8, wherein the spikes have a length smaller than that of the plurality of posts.

11. The compression staple of claim 8, wherein the body comprises a material selected from the group consisting of stainless steel, titanium and nickel titanium.

12. A compression staple, comprising:
a body having a non-linear configuration and a longitudinal axis, the body having a first surface and a second opposing surface;
two cylindrical posts located on the longitudinal axis and extending from the first surface; and
two spikes extending from the first surface, the spikes being spaced apart from each other, and being located on opposite sides relative to the longitudinal axis and to the posts.

13. The compression staple of claim 12, wherein at least a portion of the cylindrical posts is provided with ribbed barbs having undercuts for preventing pullout of the staple.
